# EUROPEAN PATENT SPECIFICATION

(11) **EP 3 302 601 B1**
(45) Date of publication and mention of the grant of the patent: **24.11.2021**
(21) Application number: 16731662.9
(22) Date of filing: 01.06.2016
(51) Int. Cl.: A61M 1/02, A61M 1/36, A61J 1/20, B65D 75/58, C12M 1/00, C12M 1/26, C12N 5/00

(54) **SYSTEM OF MULTIPLE BAGS FOR THE PREPARATION OF HEMOCOMPONENTS**
SYSTEM VON MEHREREN BEUTELN ZUR HERSTELLUNG VON HÄMOKOMPONENTEN
SYSTÈMES DE MULTIPLES SACS POUR LA PRÉPARATION D'HÉMOCOMPOSANTS

(30) Priority: 04.06.2015 IT UB20150907; 04.06.2015 IT UB20150983; 05.06.2015 US 201562171353 P
(43) Date of publication of application: 11.04.2018
(73) Proprietor: Fondazione IRCCS Ca' Granda - Ospedale Maggiore Policlinico, 20122 Milano (IT); Episkey S.r.l., 20123 Milano (IT)
(72) Inventor: MAZZARO, Giovanni, I-20123 Milano (IT); REBULLA, Paolo, I-20122 Milano (IT); PARATI, Eugenio, I-20122 Milano (IT)
(74) Representative: Croce, Valeria
(86) International application number: PCT/IB2016/053229
(87) International publication number: WO 2016/193924

(56) References cited:
- EP-A1- 1 508 345
- EP-A1- 1 579 838
- EP-A1- 1 832 522
- EP-A1- 2 500 048
- EP-A1- 2 700 707
- EP-A2- 2 304 023
- WO-A1-96/00782
- WO-A1-2011/142670
- CH-A1- 701 906
- JP-A- 2015 042 167
- US-A- 4 943 287
- US-A- 5 251 982
- US-A- 5 613 779
- US-A1- 2014 301 914
- US-A1- 2015 096 938

## Description

The present invention relates to a biomedical device for the production, storage, traceability and administration of biological products, in particular blood components, adhered onto biocompatible three-dimensional scaffolds (or substrate).

In order to prepare biocompatible scaffold (or substrate) for cell culture comprising biological material, currently sterile bags, tubes or flasks are assembled, followed by the transfer of the biological material onto the scaffold (or substrate).

Unfortunately, such a method is not free from several drawbacks, which limit its application in the therapeutic field.

In fact, it implies several procedural steps, wherein each one is inherently subject to possible errors; moreover, each handling step is a potential source of microbial contamination.

The fact that the sequence of steps is time expensive should also not be ignored.

Moreover, this procedure is not able to ensure a complete traceability of the biological product and of the devices used, which nowadays is an essential requirement of any manufacturing process, especially when applied in the medical field.

The patent application US 2014/301914 discloses an apparatus for the preparation of serum comprising an indwelling needle, a catheter, a syringe and a blood coagulation accelerating substance stored in the syringe; the document is silent on any aspect concerning the possible aperture of any one of the bags forming the system.

EP 2.500.048 discloses a blood bag system and a blood treatment method for the preparation of blood components, but does not describe that any one of the bag can be opened.

The US patent US 5.251.982 relates to a packaging container in the form of a poach; the poach comprises a channel (13'), which is formed by welding, at one of its corner regions. The channel (13') is useful for the insertion of a discharge device. In particular, the channel (13') may be opened tearing off said corner portion of the bag along two weakenings (19,20), but no means for the aperture of a bag is disclosed.

It is therefore well-known the need to develop a system for the preparation of blood components, which ensure sterility of the product and of the final products, which is compatible with large-scale production needs, which is fast, economically advantageous and which allows the entire production chain and each steps of the manufacturing process to be controlled and tracked.

### Object of the invention

The problems known in the art are thus solved by the present invention, which in a first object describes a system of multiple bags for the preparation of blood components from umbilical cord blood according to claim 1 and dependent claims.

In a second object, it is described a method for preparing blood components according to claim 15.

### Brief description of the drawings

Figure 1 shows a scheme of the system of the invention;
figure 2 shows a particular embodiment of the system of multiple bags of the invention;

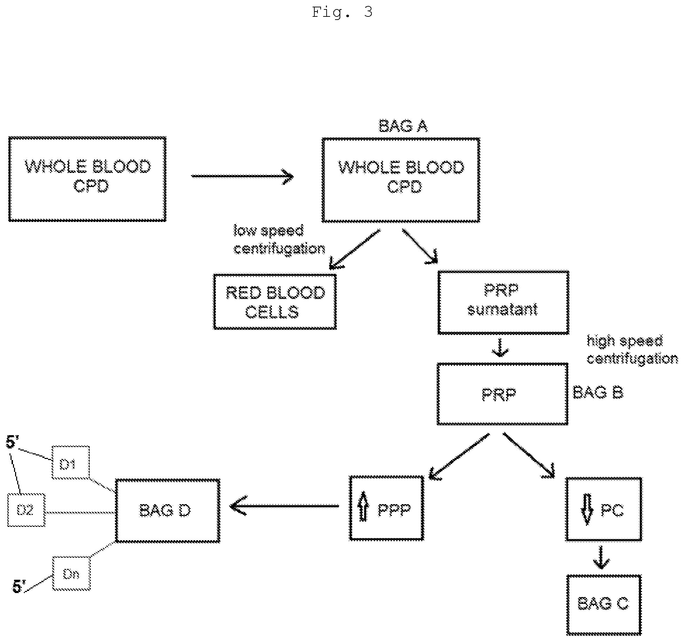

figure 3 shows a schematic representation of the process of the invention for the preparation of blood components;
figures 4A and 4B show an embodiment for bag C;
figure 5 shows another embodiment for bag C.

### Detailed description of the invention

According to the first object of the invention, it is disclosed a system of multiple bags for the preparation of blood components from blood according to claim 1 and dependent claims thereof.

In a preferred aspect, said blood component is an umbilical cord blood component.

For the present purposes, "umbilical blood component" refers to a product obtained from umbilical cord blood, and in particular it is represented by platelet-rich plasma (PRP) or platelet concentrate (PC) or platelet-poor plasma (PPP) or a culture medium for cell cultures or a platelet gel or a platelet gel activated with calcium gluconate, thrombin or batroxobin, or stem cells or plasma proteins, etc.

The application of the present patent application is to be understood to be extended also to the preparation of blood derivatives and other biological products obtainable from blood, which is not umbilical cord blood.

For simplicity and ease of reading, hereinafter reference will be made to "blood components".

According to the present description, the system of the inventions is referred to with reference 1 and comprises a bag 6 (to which reference will be made as "bag C") for the preparation of a biocompatible scaffold (or substrate) to which a blood component is adhered.

Such a blood component, in particular, is obtained with the procedure described hereinafter.

In particular, the procedure may include the use of one or more additional bags 3,4,5, so that a system of multiple bags 1 is realized.

For the present purposes, the system 1 comprises bags 3,4,5,6 suitably connected to one another, so that a fluid connection is created among them.

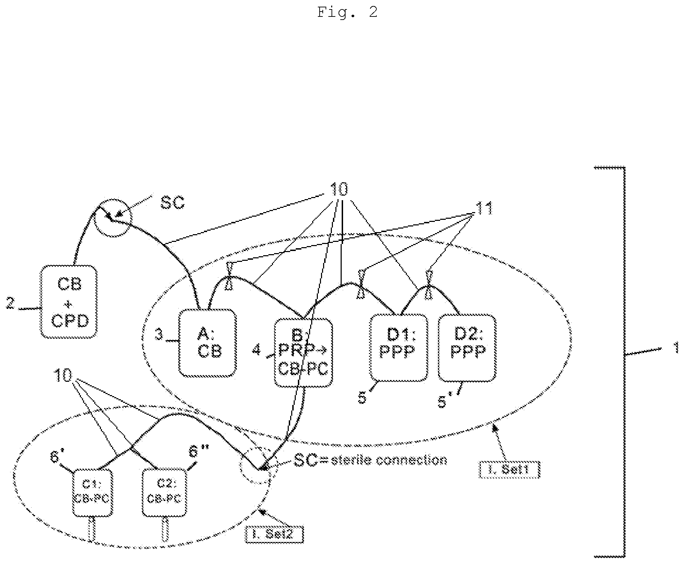

In a preferred aspect of the invention, suitable connections 10 are provided (shown in figure 2) so that it is possible to transfer the content of one bag to one or more other bags, and also to detach one or more bags from the others or, vice versa, to assemble one or more bags to the others, without damaging or interrupting the fluid communication among them and avoiding any contamination of the system and of any one of the bags.

That means that during the whole procedure for the preparation of the blood component, the sterility of system of bags and of the products contained therein is maintained, even after the detachment or assembly, and during the entire storage period.

Accordingly, the connections 10 are sterile connections (SC).

Said purpose is achieved primarily through the use of sterile bags 3,4,5,6 and appropriate sterile connections (SC) between the bags, which are capable of maintaining the sterility.

It is therefore correct to assume that not only bag C 6, but each bag 3,4,5 and the entire system 1 of the invention meet the GMP (Good Manufacturing Practices) requirements.

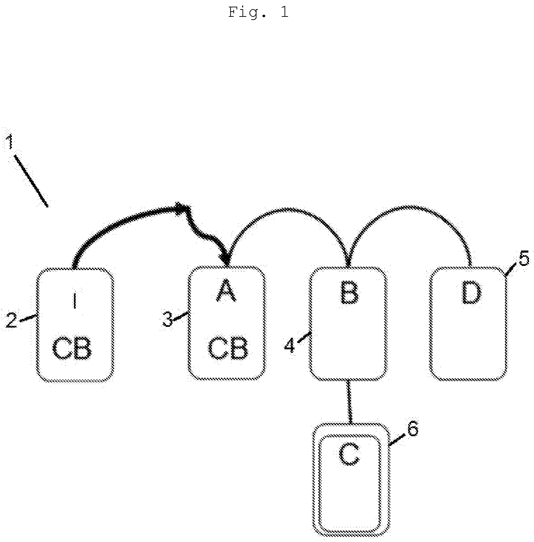

An example of the multiple bag system 1 of the invention is depicted in figures 1 and 2.

In particular it comprises:
- the bag C described above 6 for the preparation of a biocompatible scaffold (or substrate) to which a blood component adheres,
- a first bag (bag A) 3 for the separation of red blood cells (RBC) from the platelet-rich plasma (PRP);
- a second bag (bag B) 4 for the separation of a platelet sediment (pellet) and a supernatant of platelet-poor plasma (PPP), connected to bag A 3 and to the bag C 6.

In a preferred aspect of the invention, the system may further comprise:
- a bag (bag D) 5 for the separation of platelet-poor plasma (PPP), connected to bag B 4. Said bag D 5 may also be possibly connected to a plurality of single dose bags (single dose bag D1, D2, Dn or 5') for the separation of aliquots of said platelet-poor plasma (PPP).

In one embodiment of the invention, the bags 3,4,5,6 described above are also connected to a further bag 2 (bag CB) for the collection and/or the storage of a blood sample, preferably umbilical cord blood, isolated from a subject.

For the purposes of the present invention, a "subject" is intended to be a human being.

Veterinary applications involving the use of umbilical cord blood or blood which is not umbilical cord blood, from an animal are not excluded from the purposes of the present invention.

Among animals, mammals are preferred embodiments of the invention.

In a particular embodiment shown for example in figure 2, bag C 6 can be replaced by two (C1 or 6', C2 or 6") or more bags each connected to bag B 4 directly through independent connections or through a common connection in output from bag B, which is divided at each bag C1 (6') or C2 (6").

In a preferred aspect of the invention, the bag system 1 may be assembled from a kit which comprises (according to the definitions above):
- bag A (bag 3), B (bag 4), D (D1, D2 or bag 5 or 5'), which mutually pre-assembled form set 1;
- bag C (bag 6) alone or bags C1 (6'), C2 (6''), mutually pre-assembled, as set 2.

In an even more preferred aspect, such a kit further comprises bag 2.

The preparation of the kit for use requires that bag 2 is suitably connected with sets 1 and 2 above.

The connections between the three parts are obtained, as said, by means of suitable sterile connections (SC, 10), for instance in the form of connection pipes, using means known in the field of the invention, such as radio-frequency welding, which are able to ensure and maintain the sterility of the system.

The kit may further comprise suitable means for interrupting, in a reversible manner, the fluid communication between the bags 3, 4, 5, 5', 6, 6', 6" during centrifugation (as for example shown in figure 2) and thus prevent outflows of the material from one bag to another.

Such means are for example represented by clips 11 applicable to the connections 10.

As for the bags 2, 3, 4, 5, 5', 6, 6', 6" they may be represented by bags commonly employed in the medical field for the collection and storage of blood and blood components.

Therefore, the bags 2,3,4,5,5',6,6',6" may be made of PVC or other biocompatible plastic material approved for such a use and application.

In a preferred aspect, said material does not contain phthalates and, in particular, the plasticizer bis(2-ethylhexyl) phthalate (DEHP).

Moreover, at least bags A (bag 3), B (bag 4) and D (bag 5 or 5') must be made of a material resistant to centrifugations up to 3000 rpm and to temperatures up to -80°C.

In a particular aspect of the invention, the material of the inner (internal) surface of a bag 2,3,4,5,5',6,6',6" is treated with suitable anti-fouling procedures in order to prevent the formation of bacterial biofilms which could seriously compromise and impair the safety of the blood component stored within.

In another particular aspect of the invention, the material of the inner (internal) surface of a bag 2,3,4,5,6 is treated with anti-adhesive methods so as to prevent the adhesion of cells, especially platelets, which circumstance would reduce the number of platelets available for the production of the blood component.

In a preferred aspect of the invention, the material surface is subjected to suitable antithrombotic treatments in order to prevent the clumping of platelets.

Such treatments, for example, may cause surface modifications by means of plasma etching procedures, carried out with techniques and according to methods known in the field.

Moreover, bag A (bag 3) shall contains a predetermined amount of an anticoagulant agent or a mixture of agents therein.

In particular, the anticoagulant mixture comprises citrate, phosphate and dextrose (known as CPD solution).

More in detail, such a mixture may have the following composition:

| Component | Amount (g per 100 mL of anticoagulant solution) |
|---|---|
| Sodium citrate dihydrate | 2.63 |
| Sodium citrate hydrate | 0.327 |
| Monosodium phosphate dihydrate | 0.251 |
| Dextrose monohydrate | 2.55 |
| Injectable water | as needed to 100 mL |

Said anticoagulant agent or mixture of anticoagulant agents is preferably included in an amount of about 10-60% (volume/volume of blood component).

Even more preferably, said agent or mixture of agents is included in an amount of about 15 or 20 or 25 or 30 or 35 or 40 or 45 or 50 or 55% (volume/volume of blood component) and even more preferably of about 50% (volume/volume of blood component).

In one embodiment of the invention, the disclosed system of multiple bags 1 is further provided with a traceability system.

For said purpose, one or more bags 2,3,4,5,5',6,6',6'' are provided with a traceability system or device which may be represented by a microchip, a smart-code, a radio-frequency micro-transponder or one of the equivalent systems known in the art.

Preferably, the system or device used for the purposes of the present invention is represented by a micro-transponder.

In particular, such a system or device is applied to the outer wall of the bag or can be embedded in the polymer material of the bag 2,3,4,5,5',6,6',6".

The traceability system or device allows to record and store several data about the bag 2,3,4,5,5',6,6',6", its production process (batch, production date) and the production process of the blood component and patient to whom the product is assigned.

From the data stored in the traceability system or device it is thus possible to trace the entire production and distribution process of the bag system 1 even after the delivery to hospitals, pharmacies or patients.

According to claim 1, the bags 6,6',6" of the system 1 further comprise means for the facilitated opening of the bag itself.

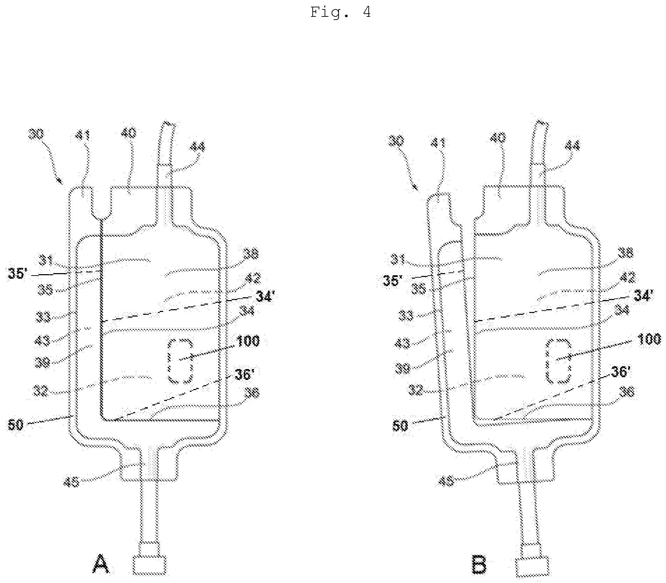

Figure 4 shows an embodiment of bag C (referred to as 30), which is defined, as other bags used for similar purposes, by an upper sheet 31 and lower sheet 32 made of a suitable polymeric material, the two sheets 31,32 being mutually coupled to the outer perimeter 33 by means of a suitable welding ("full" welding).

Said suitable welding of the two sheets 31 and 32 creates an edge 50.

In the embodiment of the invention, bag C (6,6',6",30) contains a scaffold (or substrate) 100 according to the disclosure here below.

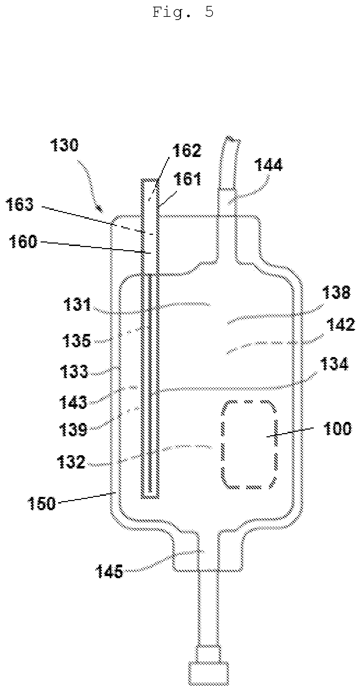

For instance, figures 4 and 5 show the presence of such a scaffold (or substrate) 100 within bag C (6,6',6",30).

Bag C (6,6',6",30) is connected to the system 1 of the invention through appropriate in 44 and out 45 manifold tubes.

According to the invention, the upper sheet 31 of bag C (6,6',6",30) comprises a facilitated breakage portion 34 for the same sheet 31.

Also the lower sheet 32 comprises a facilitated breakage portion 34'.

In one embodiment of the invention, such a facilitated breakage portion 34,34' comprises a partial engraving in the thickness of the sheet 31 and/or 32 (that means that the thickness of the polymeric material of the sheet is partially cut), which is obtained by welding-and-engraving techniques known in technical field.

In other words, the facilitated breakage portion 34,34' may be represented by one or a plurality of partial engravings in the thickness of the sheet 31 and/or 32.

In other embodiments, the facilitated breakage portion 34,34' may comprise of one or more, rectilinear or curvilinear engraving realized on one or on both sheets 31,32.

Obviously, such a welding-and-engraving is realized by suitable techniques capable to keep the sterility of the bag and its content and, therefore, of the entire system according to the present invention.

According to one embodiment of the invention shown for example in figure 4B, a facilitated breakage portion 34,34' may comprises a longitudinal engraving portion 35,35' (along the larger dimension of the bag) and a transversal engraving portion 36,36' (along the shorter dimension of the bag).

The facilitated breakage portion 34,34' of the present invention allows to divide sheet 31 and/or 32 of bag C (6,6',6",30) in two portions: a fixed portion 38,42 and an opening portion 39,43.

A complete opening of bag C 6,6',6",30 (or of any bag provided with said facilitated breaking portion) advantageously allows the facilitated extraction of the content from bag C 6,6',6",30.

The complete opening allows a method of extraction of the content, which is quick and safe, thus preventing possible contamination of the content by the operator, with their hands or gloves.

Moreover, in one embodiment of the invention, auxiliary opening means of the bag C 6,6',6",30 may also be provided, especially for the manual opening. Said means may represented by one or more gripping portions 40 which allow the fixed portions 38,42 to be retained with one hand by the operator.

Advantageously, this allows to exert a certain retention force and, therefore, a more convenient and easy opening of bag C 6,6',6",30.

In one aspect of the invention, such means are coupled to the fixed portions of the upper 38 and/or lower 42 sheet of the bag C 6,6',6",30.

In a preferred embodiment, the auxiliary opening means 40 are close to the facilitated opening portion 34,34' and possibly close to the in manifold pipe 44.

Such auxiliary opening means are for example represented by the tab 40 shown in figures 4A and 4B.

In one embodiment of the invention, said auxiliary opening means 40 may represented by a suitable portion of the edge 50 of the bag C 6,6',6",30.

The bag C 6,6',6",30 of the invention may further comprise additional auxiliary opening means.

Such additional auxiliary opening means may represented by one or more gripping portions 41 which allow to grip and hold the opening portions 39,43 of bag C 6,6',6",30.

Advantageously, these additional auxiliary opening means allow to exert a tearing force on the facilitated opening portion 34,34' and, therefore, render an even more comfortable and easy opening of bag C 6,6',6",30.

In a particular aspect, such additional auxiliary opening means are coupled to the opening portion of the upper and/or lower sheets 39,43.

In a preferred embodiment, the additional auxiliary opening means are close to the facilitated breakage portion 34.

Such additional auxiliary opening means are for example represented by tab 41 shown in figures 4A and 4B.

In one embodiment of the invention, said additional auxiliary opening means 41 are represented by a suitable portion of the edge 50 of the bag C 6,6',6",30.

According to an alternative embodiment of the invention which is represented in figure 5, the bag C 6,30,130 (or any other bag of the system) may comprise elements 160 for the facilitated opening of the bag 6,30,130.

For said purposes, the bag C 6,30,130 shown in figure 5 is defined by an upper sheet 131 and lower sheet 132 of a suitable polymeric material, the two sheets 131,132 being mutually coupled to the outer perimeter 133 by means of a suitable welding ("full" welding) .

Said suitable welding of the two sheets 131 and 132 creates an edge 150.

Inside the bag C 6,30,130 there is contained the scaffold (or substrate) 100 according to the present invention.

Bag C 6,30,130 is connected to the system 1 of the invention through appropriate in 144 and out 145 manifold tubes.

As per claim 1, the upper sheet 131 of bag C 6,6',6",30 comprises a facilitated breaking portion 134 of the same sheet 131.

As per claim 1, also the lower sheet 132 of the bag C 6,6',6",30 comprises a facilitated breaking portion 135 of the same sheet 132.

According to one embodiment of the invention, said facilitated breaking portion 134,135 comprises a partial engraving in the thickness of the sheet 131 and/or 132 (that means that the thickness of the polymeric material of the sheet is partially cut), which is obtained by welding-and-engraving techniques known in the technical field.

According to the present invention, the upper sheet 131 of bag C 6,30,130 may comprise elements for the facilitated breaking and opening of the bag C 160.

In an alternative embodiment, also the lower sheet 132 comprises elements 163 for the facilitated opening of the bag C.

As per claim 1, the elements for the facilitated breaking and opening of the bag 160,163 allows to easily divide sheet 131 and/or 132 of bag C 6,6',6",30,130 into two portions: a fixed portion 138,142 and an opening portion 139,143.

Preferably, said elements for the facilitated breaking and opening of the bag 160,163 is suitably welded in correspondence with the facilitated breaking portion 134 and/or 135 (as shown in figure 5).

In a preferred aspect, said elements for the facilitated opening of the bag 160,163 may be represented by a peel-off system.

Said elements for the facilitated opening of the bag 160,163 can be manually actuated with a gripping element, for example represented by a tab 161,162 on each sheet of the bag 131,132.

In one alternative embodiment, the elements for the facilitated opening of the bag 160,163 may comprise a rupture element (not shown in the figures) embedded in the polymeric material of the sheet 131 and/or 132 capable of cutting the polymeric sheet 131 and/or 132.

According to a preferred aspect of the invention, the bag C 6,6',6",30,130 of the system 1 comprises a scaffold (or substrate) 100 to which a blood component can adhere.

In a preferred aspect of the invention, the blood component is represented by a platelet concentrate (PC) or a derivative of platelets, such as a platelet gel.

With reference to the scaffold (or substrate) 100 of the invention, it is represented by a matrix, possibly a biocompatible matrix.

For said purpose, a material can be used among:
- materials of proteic origin,
- non proteic polisaccharides,
- non degradable synthetic polymers,
- degradable polymers,
- other materials.

In particular:
- material of proteic origin may include: fibroin, collagen, gelatin, retronectin and other similar materials;
- non proteic polisaccharides may include: chitosan, hyaluronic acid, alginates, ulvan and other similar materials;
- non degradable synthetic polymers may include: polyester, hydrogels and other similar materials;
- degradable polymers may include: polylactic acid, polyglycolic acid, polycaprolactone and other similar materials.

Other materials that may be used as scaffold (or substrate) 100 include: ceramic (hydroxyapatite).

In a preferred aspect, the material of scaffold (or substrate) 100 is fibroin.

According to a preferred embodiment, the scaffold (or substrate) 100 can be subjected to sterilization.

According to a preferred embodiment, the scaffold (or substrate) 100 is not allergenic (capable of eliciting an allergic reaction).

In a particular aspect of the invention, such a scaffold (or substrate) 100 is obtained by means of the 3D printer technology.

According to one embodiment of the invention, the scaffold (or substrate) 100 may be in the form of a patch; alternatively, it may have a cylindrical shape, such as cigarette, suppository, or it may be made in any other appropriate three-dimensional shape suitable for specific therapeutic needs.

In fact, in a particular aspect, the invention discloses the preparation of a shaped scaffold (or substrate) 100 capable of filling a predetermined body cavity, such as a bone cavity.

The information needed to produce a scaffold (or substrate) 100 having precise size and shape may be obtained by means of diagnostic imaging techniques, such as computed tomography.

This allows to create a special-purpose product to meet the needs of a specific patient, as part of the so-called "personalized medicine".

According to an alternative embodiment of the invention, the scaffold (or substrate) 100 for the platelet gel is not represented by an element but it is represented by the inner (internal) surface of the bag itself (it can be considered itself as a patch).

In a preferred embodiment, said inner surface is the inner (internal) surface of bag C 6,6',6",30,130.

To this end, the inner (internal) surface is suitably treated with plasma procedures employing a gas of a different nature (oxygen, argon, hydrogen, nitrogen, air, etc.) in order to increase the platelet adhesion.

The plasma treatment is preferably carried out at room temperature (cold plasma) and at atmospheric pressure.

Moreover, such a treatment is conducted under conditions preventing and avoiding any microbial contamination of the substrate.

Once opened, two elements (two patches) can advantageously be obtained from the bag C 6,6',6",30,130, each comprising part of the bag wall and a portion of platelet gel adhered thereto.

The present invention therefore discloses a medical device represented by a patch obtained with the use of a bag comprising a scaffold (or substrate) which scaffold (or substrate) may also consist of the inner surface of the bag itself, to which a blood component, in particular a platelet gel, has adhered to.

Therefore, here disclosed are: a bag for blood components comprising a scaffold (or substrate) to which a blood component, in particular a platelet gel, has adhered, a bag for blood components comprising a blood component adhered to the inner surface of the bag itself, both bags being or not provided with a facilitated opening system as one of the systems above described and a multiple bag system comprising each of said bags.

According to a further aspect of the present invention, the bag C 6,6',6",30,130 described above, alone or as a part of the bag system disclosed, comprising a biocompatible scaffold (or substrate) according to the definition given above, wherein said scaffold (or substrate) can also be represented by the inner surface of the bag itself, and wherein a blood component has not adhered to said scaffold (or substrate), may be used as a cell culture chamber.

In a preferred aspect, such cells are stem cells.

According to a second object, the present invention describes a method for preparing blood components through the use of the system of multiple bags described herein according to claim 15.

Such a procedure comprises the treatment of an isolated blood sample, preferably umbilical cord blood, collected in a first bag 2.

Inside bag 2, in particular, the sample is contacted with an anticoagulant preparation represented, for example, by a mixture comprising citrate, phosphate and dextrose (known as CPD solution).

More in particular, such a mixture may have the following composition:

| Component | Amount (g per 100 mL of anticoagulant solution) |
|---|---|
| Sodium citrate dihydrate | 2.63 |
| Sodium citrate hydrate | 0.327 |
| Monosodium phosphate dihydrate | 0.251 |
| Dextrose monohydrate | 2.55 |
| Injectable water | as needed to 100 mL |

Said anticoagulant preparation is included in an amount of about 10-60% (volume/volume of blood component).

Preferably, said preparation is included in an amount of about 15 or 20 or 25 or 30 or 35 or 40 or 45 or 50 or 55% (volume/volume of blood component) and even more preferably of about 50% (volume/volume of blood component).

According to a first step, the blood collected in bag 2 is transferred to a bag A (bag 3) which is subjected to a low-speed centrifugation, obtaining at the end a sediment at the bottom of the bag represented by red blood cells, while the platelet-rich plasma (PRP) is separated as a surnatant.

In particular, such a step is carried out at a speed of about 220 rpm and for a period of about 10 minutes.

In a second step, the sediment of red blood cells is separated from the platelet-rich plasma, which is transferred to a second bag (bag B, bag 4).

In a third step, bag B (bag 4) is subjected to high-speed centrifugation in order to concentrate the platelets in a small volume at the bottom.

Such a step is preferably carried out at a speed of about 2,000 rpm and for a period of about 15 minutes.

The supernatant obtained is represented by platelet-poor plasma (PPP) which is transferred to another bag (bag D, bag 5), while the platelet concentrate (PC) is kept in bag B (bag 4).

In a subsequent step, the excess platelet-poor plasma (5-10 mL) is transferred in a further bag (bag D) .

Bag D (bag 5) is then separated from the multiple bag system 1.

According to a preferred aspect of the invention, bag D (bag 5) is connected to a plurality of single-dose bags (D1, D2, Dn, generally referred to as 5') for the storage and the administration of platelet-poor plasma aliquots (CBPPP).

In a preferred aspect, such aliquots have a volume of about 1 mL.

In particular, the single doses of CBPPP are used as eye drops.

As regards the platelet concentrate (PC) in a volume of about 5-10 mL collected in bag B (bag 4), in a subsequent step it is suspended and transferred into bag C (bag 6).

Water or saline is preferably used for the suspension of the platelets.

According to a preferred aspect of the invention, bag C (bag 6) comprises internally a three-dimensional scaffold (or substrate) to which the platelets adhere.

Said scaffold (or substrate) is preferably the scaffold (or substrate) above disclosed.

The activation of the sediment of platelets by means of calcium gluconate, thrombin or batroxobin leads to the formation of a platelet gel (CBPG).

The material of bag C (bag 6) is suitably selected so that it can then be subjected to freezing at a temperature of -90°C/-70°C and preferably of -80°C and thus stored.

Alternately, the gel may be subjected to freeze-drying which, when needed, can be reconstituted by the addition of water or saline.

During the steps of the procedure described, the fluid communication between the bags 3,4,5,6 may be suitably (reversibly) interrupted with suitable means, (such as shown in Figure 2) in order to prevent outflows of material between them.

In one embodiment, such means are represented by appropriate clips 11.

As for the scaffold (or substrate), it is represented by a biocompatible matrix.

Therefore, the following materials can be used:
- materials of proteic origin,
- non proteic polisaccharides,
- non degradable synthetic polymers,
- degradable polymers,
- other materials.

In particular:
- material of proteic origin may include: fibroin, collagen, gelatin, retronectin and other similar materials;
- non proteic polisaccharides may include: chitosan, hyaluronic acid, alginates, ulvan and other similar materials;
- non degradable synthetic polymers may include: polyester, hydrogels and other similar materials;
- degradable polymers may include: polylactic acid, polyglycolic acid, polycaprolactone and other similar materials.

Other materials that may be used as scaffold (or substrate) include: ceramic (hydroxyapatite).

In a preferred aspect, the material of scaffold (or substrate) is fibroin.

According to a preferred embodiment, the scaffold (or substrate) can be subjected to sterilization.

According to a preferred embodiment, the scaffold (or substrate) is not allergenic (capable of eliciting an allergic reaction).

In a particular aspect of the invention, such a scaffold (or substrate) is prepared through the 3D printer technology.

According to one embodiment of the invention, the scaffold (or substrate) may be in the form of a patch; alternatively, it may have a cylindrical shape, such as cigarette, suppository, or it may be made in any other appropriate three-dimensional shape suitable for specific therapeutic needs.

In fact, a particular aspect includes preparing a shaped scaffold (or substrate) so as to fill a predetermined cavity, such as a bone cavity.

The information needed to produce a scaffold (or substrate) having precise size and shapes may be obtained by means of diagnostic imaging techniques, such as computed tomography.

This allows to create a special-purpose product to meet the needs of a specific patient, as part of the so-called "personalized medicine".

According to an alternative embodiment of the invention, the scaffold (or substrate) for the platelet gel is represented by the inner surface of one of the bag of the system 1 of the invention.

In particular, the inner (internal) surface of the bag C (bag 6,6',6") may be used.

To this end, the surface is suitably treated with plasma procedures employing a gas of a different nature (oxygen, argon, hydrogen, nitrogen, air, etc.) in order to increase the platelet adhesion.

The plasma treatment is preferably carried out at room temperature (cold plasma) and at atmospheric pressure.

Such a treatment is conducted so as to prevent and avoid any microbial contamination.

According to one aspect of the invention, the preparation of a blood component from umbilical cord blood is preceded by a step of selection of the isolated blood samples to verify the total nucleated cell counts as an approximation of the content of the hematopoietic stem cells suitable for transplantation and possibly, testing for markers for syphilis, HIV, HCV, HBV, bacteria, fungi.

Therefore, in one aspect of the invention, the umbilical cord blood samples used in the preparation of the blood component described are those samples which do not meet the conditions required for the application in the treatment of blood diseases.

According to a further aspect, a biocompatible scaffold (or substrate) is described to which a blood component, preferably a platelet gel, has adhered inside a bag under complete asepsis conditions.

According to an object which is not within the purposes of the present invention, the use of the medical devices obtained according to the present invention for medical treatment is described.

In particular, such a treatment relates to skin ulcers and decubitus ulcers and corneal diseases such as: dry eye syndrome, graft-versus-host disease (GVHD), injuries caused by chemical burns, neurotrophic keratitis, Sjogren's syndrome, systemic sclerosis, rheumatoid arthritis and autoimmunity, corneal ulcers, keratoconjunctivitis.

For the purposes of the present disclosure, such devices are represented by the bag, the bag system, the patch and the scaffold (or substrate) bearing the platelet gel.

The several advantages offered by the present invention will be apparent from the description above.

First, the system allows to apply a method for simultaneously obtaining two important blood components: a platelet-poor plasma and a platelet gel.

As regards the first product, this can be used in place of synthetic preparations used to treat certain corneal diseases: the so-called "artificial tears".

Moreover, while there are eye drop preparations obtained from autologous blood serum, these are not without drawbacks and contraindications.

In fact, on the one hand, the patient's blood collection is required, which procedure is not always easy in children and elderly people.

Moreover, while autologous serum offers potential advantages with respect to allogeneic products (compatibility and low risk of transmission of pathogens), it presumably exposes the patients themselves to altered biologic mediators related to their disease.

Therefore, the preparation of allogeneic eye drops is a great opportunity for an alternative use of the scrap units of placental blood, which in about 80-90% of cases is not suitable for use in transplantation in severe blood diseases.

On the other hand, as regards the use of the platelet gel, this can be included in the treatment protocols of skin ulcers and decubitus ulcers, for which the present invention provides a solution which meets current and future therapeutic needs of the health service.

In fact, recent data indicate that about 3% of the population in advanced economies countries is suffering from chronic skin lesions and sores.

A recent evaluation done in Italy (ADNKronos Salute, March 6, 2014) indicates that about 4% of the total costs of the national health system is dedicated to the treatment of about 2 million patients suffering from these diseases, with an absolute cost of nearly 1 billion euros per year.

These costs are generated by 15-20% by the purchase of materials, by 30-35% by the time of the nursing staff and by the remaining 50% by the hospitalization of patients.

Other data from American hospitals indicate that the occurrence of pressure injuries can prolong hospital stays up to 5 times, with an expenditure increase of $ 2,000-11,000 per patient.

The prevention and early treatment of these injuries not only offers the opportunity to reduce morbidity and mortality in these patients, but also significant savings of economic resources.

In addition to reducing costs, a shorter hospital stay almost always is preferred by the patients themselves.

Moreover, it was surprisingly found that a blood component, in particular a platelet gel, adhered to a biocompatible scaffold (or substrate), and especially fibroin, has greater stability over time, retaining its therapeutic properties for a longer time.

An important advantage provided by the multiple bag system of the invention and by the method for preparing blood components and scaffold (or substrate) bearing blood components is the ability to carry out the entire procedure under aseptic conditions and in compliance with the Good Manufacturing Practices (GMP).

Moreover, the facilitated opening bag allows to store the platelet gel adhered to the scaffold (or substrate) under sterile conditions and therefore is a decidedly practical system for transport and distribution of the same until administration to the patient.

Those skilled in the art will be able to make changes and adaptations to the present invention, without however departing from the scope of the following claims.

## Claims

1. A multiple bag system (1) for the preparation and storage of blood components comprising a bag (6) for the adhesion of a blood component product to a biocompatible scaffold (100),
- a bag (3) for the separation of red blood cells from the platelet-rich plasma;
- a bag (4) for the separation of a platelet sediment and a supernatant of platelet-poor plasma, connected to said bag (3) for the separation of red blood cells from the platelet-rich plasma and to said bag (6) for the adhesion of the blood component product,
- a bag (5) for the separation of said platelet-poor plasma, connected to said bag for the separation of a platelet sediment and a supernatant of platelet-poor plasma (4), and possibly a plurality of further bags (5') for the separation of aliquots of platelet-poor plasma connected to said bag (5) for the separation of said platelet-poor plasma,
wherein said bag (6,6',6",30,130) for the adhesion of the blood component product comprises an upper sheet (31,131) and a lower sheet (32,132) shaped and mutually coupled to the perimeter (33,133) by means of suitable welding thus originating an edge (50,150), and **characterized in that** said upper sheet (31,131) and/or said lower sheet (32,132) comprises a facilitated breakage portion (34,134) of the sheet itself (31,131 32,132) and wherein the facilitated breakage portion (34,134) divides said upper sheet (31,131) and/or said lower sheet (32,132) in an opening portion (39,43) and a fixed portion (38,42) thus allowing a complete opening of the bag (6) for the adhesion of the blood component product to allow the facilitated extraction of the content from the bag (6) for the adhesion of the blood component product and wherein said bag (6) for the adhesion of the blood component product, comprises means for the facilitated opening of the bag itself.

2. The bag system (1) according to the preceding claim, further comprising a bag (2) for collecting an isolated whole blood sample from a subject.

3. The bag system (1) according to any one of the preceding claims, wherein said bag (6) for the adhesion of said blood component product to a biocompatible scaffold (100) may be replaced by two or more bags (6', 6").

4. The multiple bag system (1) according to any one of the preceding claims, wherein said bags (2,3,4,5,5',6,6',6") are mutually connected by means of sterile connections (10) capable of maintaining sterility of the system (1).

5. The multiple bag system (1) according to any one of the preceding claims, wherein said bag (6,6',6") for the adhesion of said blood component product to a biocompatible scaffold (100) comprises a biocompatible material scaffold contained therein.

6. The multiple bag system (1) according to the preceding claim, wherein said biocompatible material of the scaffold 100 is selected from the group comprising:
- material of proteic origin, which may include: fibroin, collagen, gelatin, retronectin and other similar materials;
- non proteic polisaccharides, which may include: chitosan, hyaluronic acid, alginates, ulvan and other similar materials;
- non degradable synthetic polymers, which may include: polyester, hydrogels and other similar materials;
- degradable polymers, which may include: polylactic acid, polyglycolic acid, polycaprolactone and other similar materials;
- other materials that may include: ceramic (hydroxyapatite).

7. The multiple bag system (1) according to any one of the preceding claims 1 to 4, wherein said support is represented by the inner surface of the bag (6,6',6") for the adhesion of the blood component product.

8. The multiple bag system (1) according to any one of the preceding claims, wherein said bags (2,3,4,5,5',6,6',6") are made of a material which does not comprise bis(2-ethylhexyl) phthalate *(DEHP).*

9. The multiple bag system (1) according to any one of the preceding claims, wherein the inner surface of said bags (2,3,4,5,5',6,6',6") is modified by means of anti-fouling and/or anti-adhesion and/or antithrombotic treatments.

10. The multiple bag system (1) according to claim 1, wherein said facilitated breakage portion (34,134) comprises one or more partial engraving (35,36,35',36') of the thickness of said sheet (31,131 and/or 32,132).

11. The multiple bag system (1) according to the preceding claim, wherein said engraving (35,36,35',36') are mutually continuous.

12. The multiple bag system (1) according to any one of the preceding claims, wherein said bag (6,6',6",30,130) for the adhesion of the blood component product further comprises auxiliary means for the bag opening.

13. The multiple bag system (1) according to the preceding claim, wherein said auxiliary means comprise one or more gripping portions (40) to exert a retention force on the facilitated opening portion (34,134).

14. The multiple bag system (1) according to any one of the preceding claims 12 or 13, wherein said auxiliary means are coupled to the fixed portions of the upper (38,138) and/or lower (42,142) sheets.

15. A method for preparing blood components, comprising the use of the multiple bag system (1) according to any one of the preceding claims, comprising the steps of:
a) subjecting an isolated sample of umbilical cord blood comprised in the bag (3) for the separation of red blood cells from the platelet-rich plasma to a low-speed centrifugation carried out at a speed of about 220 rpm for a period of about 10 minutes, thus obtaining a sediment represented by red blood cells and a supernatant represented by platelet-rich plasma (PRP);
b) transferring said plasma to the bag (4) for the separation of a platelet sediment and a supernatant of platelet-poor plasma;
c) subjecting said plasma in the bag (4) for the separation of a platelet sediment and a supernatant of platelet-poor plasma to a high-speed centrifugation carried out at a speed of about 2,000 rpm for a period of about 15 minutes, thus obtaining platelet-poor plasma (PPP) and a platelet concentrate (PC);
d) transferring said platelet-poor plasma (PPP) to the bag (5) for the separation of said platelet-poor plasma;
e) transferring said platelet concentrate (PC) to a the bag (6) for the adhesion of a blood component product to a biocompatible scaffold (100).

## Patentansprüche

1. Mehrfachbeutelsystem (1) zur Herstellung und Lagerung von Blutkomponenten, umfassend einen Beutel (6) zum Anhaften eines Blutkomponentenprodukts an ein biokompatibles Gerüst (100),
- einen Beutel (3) zur Abtrennung roter Blutzellen aus dem plättchenreichen Plasma;
- einen Beutel (4) zur Abtrennung eines Plättchensediments und eines Überstands von einem plättchenarmen Plasma, der mit dem Beutel (3) zur Abtrennung von roten Blutzellen aus dem plättchenreichen Plasma und mit dem Beutel (6) für die Anhaftung des Blutkomponentenprodukts verbunden ist,
- einen Beutel (5) zur Abtrennung des plättchenarmen Plasmas, der mit dem Beutel (4) zur Abtrennung eines Plättchensediments und eines Überstands des plättchenarmen Plasmas verbunden ist, und gegebenenfalls eine Mehrzahl weiterer Beutel (5') zur Abtrennung von Aliquots von plättchenarmen Plasma, die mit dem Beutel (5) zur Abtrennung des plättchenarmen Plasmas verbunden sind,
wobei der Beutel (6, 6', 6", 30, 130) für die Anhaftung des Blutkomponentenprodukts eine obere Folie (31, 131) und eine untere Folie (32, 132), geformt und gegenseitig über den Umfang (33, 133) durch geeignetes Schweißen verbunden, umfasst, wodurch eine Kante (50, 150) entsteht, und **dadurch gekennzeichnet, dass** die obere Folie (31, 131) und/oder die untere Folie (32, 132) einen erleichterten Bruchabschnitt (34,134) der Folie (31, 131 32, 132) selbst umfasst/umfassen und wobei der erleichterte Bruchabschnitt (34, 134) die obere Folie (31, 131) und/oder die untere Folie (32, 132) in einen Öffnungsabschnitt (39, 43) und einen festen Abschnitt (38, 42) trennt, wodurch ein komplettes Öffnen des Beutels (6) zur Anhaftung des Blutkomponentenprodukts ermöglicht wird, um die erleichterte Entnahme des Inhalts aus dem Beutel (6) zur Anhaftung des Blutkomponentenprodukts zu ermöglichen und wobei der Beutel (6) zur Anhaftung des Blutkomponentenprodukts Mittel zum erleichterten Öffnen des Beutels selbst umfasst.

2. Beutelsystem (1) nach dem vorhergehenden Anspruch, ferner umfassend einen Beutel (2) zum Sammeln einer isolierten Vollblutprobe von einem Individuum.

3. Beutelsystem (1) nach einem der vorhergehenden Ansprüchen, wobei der Beutel (6) zum Anhaften des Blutkomponentenprodukts an ein biokompatibles Gerüst (100) durch zwei oder mehr Beutel (6', 6") ersetzt werden kann.

4. Mehrfachbeutelsystem (1) nach einem der vorhergehenden Ansprüche, wobei die Beutel (2, 3, 4, 5, 5', 6, 6', 6") mittels steriler Verbindungen (10), die dazu eingerichtet sind, die Sterilität des Systems (1) aufrechtzuerhalten, miteinander verbunden sind.

5. Mehrfachbeutelsystem (1) nach einem der vorhergehenden Ansprüche, wobei der Beutel (6, 6', 6") zum Anhaften des Blutkomponentenprodukts an ein biokompatibles Gerüst (100) ein darin enthaltenes biokompatibles Materialgerüst umfasst.

6. Mehrfachbeutelsystem (1) nach dem vorhergehenden Anspruch, wobei das biokompatible Material des Gerüsts 100 ausgewählt ist aus der Gruppe umfassend:
- Material proteinösen Ursprungs, das umfassen kann: Fibroin, Kollagen, Gelatine, Retronectin und andere ähnliche Materialien;
- nicht proteinöse Polysaccharide, die umfassen können: Chitosan, Hyaluronsäure, Alginate, Ulvan und andere ähnliche Materialien;
- nicht abbaubare synthetische Polymere, die umfassen können: Polyester, Hydrogele und andere ähnliche Materialien;
- abbaubare Polymere, die umfassen können: Polymilchsäure, Polyglycolsäure, Polycaprolacton und andere ähnliche Materialien;
- andere Materialien, die umfassen können: Keramik (Hydroxyapatit).

7. Mehrfachbeutelsystem (1) nach einem der vorhergehenden Ansprüche 1 bis 4, wobei der Träger durch die Innenfläche des Beutels (6, 6', 6") zum Anhaften des Blutkomponentenprodukts dargestellt wird.

8. Mehrfachbeutelsystem (1) nach einem der vorhergehenden Ansprüche, wobei die Beutel (2, 3, 4, 5, 5', 6, 6', 6") aus einem Material hergestellt sind, das kein Bis(2-ethylhexyl)phthalat (DEHP) umfasst.

9. Mehrfachbeutelsystem (1) nach einem der vorhergehenden Ansprüche, wobei die Innenfläche der Beutel (2, 3, 4, 5, 5', 6, 6', 6") modifiziert ist mittels Anti-Fouling- und/oder Anti-Adhäsions- und/oder Anti-Thrombose-Behandlungen.

10. Mehrfachbeutelsystem (1) nach Anspruch 1, wobei der erleichterte Bruchabschnitt (34, 134) eine oder mehrere Teilgravuren (35, 36, 35', 36') der Dicke der Folie (31, 131 und/oder 32,132) umfasst.

11. Mehrfachbeutelsystem (1) nach dem vorhergehenden Anspruch, wobei die Gravuren (35, 36, 35', 36') gegenseitig kontinuierlich sind.

12. Mehrfachbeutelsystem (1) nach einem der vorhergehenden Ansprüche, wobei der Beutel (6,6',6", 30, 130) zum Anhaften des Blutkomponentenprodukts ferner Hilfsmittel zum Öffnen des Beutels umfasst.

13. Mehrfachbeutelsystem (1) nach dem vorhergehenden Anspruch, wobei die Hilfsmittel einen oder mehrere Greifabschnitte (40) umfassen, um eine Haltekraft auf den erleichterten Öffnungsabschnitt (34, 134) auszuüben.

14. Mehrfachbeutelsystem (1) nach einem der vorhergehenden Ansprüche 12 oder 13, wobei die Hilfsmittel mit den festen Abschnitten der oberen (38, 138) und/oder unteren (42, 142) Folien gekoppelt sind.

15. Verfahren zur Herstellung von Blutkomponenten, umfassend die Verwendung des Mehrfachbeutelsystems (1) nach einem der vorhergehenden Ansprüche, umfassend die Schritte:
a) Unterziehen einer isolierten Nabelschnurblutprobe, die in dem Beutel (3) zur Abtrennung roter Blutzellen aus dem plättchenreichen Plasma enthalten ist, einer Zentrifugation bei niedriger Geschwindigkeit, durchgeführt bei einer Geschwindigkeit von etwa 220 Umdrehungen pro Minute für einen Zeitraum von etwa 10 Minuten, wodurch ein durch rote Blutzellen repräsentiertes Sediment und ein durch plättchenreiches Plasma (PRP) repräsentierter Überstand erhalten werden;
b) Übertragen des Plasmas in den Beutel (4) zur Abtrennung eines Plättchensediments und eines Überstands von einem plättchenarmen Plasma;
c) Unterziehen des Plasmas in Beutel (4) zur Abtrennung eines Plättchensediments und eines Überstands von einem plättchenarmen Plasma einer Zentrifugation bei hoher Geschwindigkeit, durchgeführt bei einer Geschwindigkeit von etwa 2000 Umdrehungen pro Minute für einen Zeitraum von etwa 15 Minuten, wodurch plättchenarmes Plasma (PPP) und ein Plättchenkonzentrat (PC) erhalten werden;
d) Überführen des plättchenarmen Plasmas (PPP) in den Beutel (5) zur Abtrennung des plättchenarmen Plasmas;
e) Überführen des Plättchenkonzentrats (PC) in den Beutel (6) zum Anhaften eines Blutkomponentenprodukts an ein biokompatibles Gerüst (100).

## Revendications

1. Système de multiples sacs (1) pour la préparation et le stockage de composants sanguins comprenant un sac (6) pour l'adhérence d'un produit de composant sanguin à un support biocompatible (100),
- un sac (3) pour la séparation de globules rouges du plasma riche en plaquettes ;
- un sac (4) pour la séparation d'un sédiment plaquettaire et d'un surnageant de plasma pauvre en plaquettes, connecté audit sac (3) pour la séparation de globules rouges du plasma riche en plaquettes et audit sac (6) pour l'adhérence du produit de composant sanguin,
- un sac (5) pour la séparation dudit plasma pauvre en plaquettes, connecté audit sac pour la séparation d'un sédiment plaquettaire et d'un surnageant de plasma pauvre en plaquettes (4), et possiblement une pluralité de sacs (5') supplémentaires pour la séparation d'aliquotes de plasma pauvre en plaquettes connectés audit sac (5) pour la séparation dudit plasma pauvre en plaquettes,
dans lequel ledit sac (6, 6', 6", 30, 130) pour l'adhérence du produit de composant sanguin comprend une feuille supérieure (31, 131) et une feuille inférieure (32, 132) façonnées et mutuellement couplées au périmètre (33, 133) au moyen d'un soudage approprié créant ainsi un bord (50, 150), et **caractérisé en ce que** ladite feuille supérieure (31, 131) et/ou ladite feuille inférieure (32, 132) comprend une partie de rupture facilitée (34, 134) de la feuille elle-même (31, 131 32, 132) et dans lequel la partie de rupture facilitée (34, 134) divise ladite feuille supérieure (31, 131) et/ou ladite feuille inférieure (32, 132) en une partie d'ouverture (39, 43) et une partie fixe (38, 42) permettant ainsi une ouverture complète du sac (6) pour l'adhérence du produit de composant sanguin pour permettre l'extraction facilitée du contenu du sac (6) pour l'adhérence du produit de composant sanguin et dans lequel ledit sac (6) pour l'adhérence du produit de composant sanguin, comprend un moyen pour l'ouverture facilitée du sac lui-même.

2. Système de sacs (1) selon la revendication précédente, comprenant en outre un sac (2) pour collecter un échantillon isolé de sang entier à partir d'un sujet.

3. Système de sacs (1) selon l'une quelconque des revendications précédentes, dans lequel ledit sac (6) pour l'adhérence dudit produit de composant sanguin à un support biocompatible (100) peut être remplacé par deux sacs (6', 6") ou plus.

4. Système de multiples sacs (1) selon l'une quelconque des revendications précédentes, dans lequel lesdits sacs (2, 3, 4, 5, 5', 6, 6', 6") sont mutuellement connectés au moyen de connexions stériles (10) capables de maintenir la stérilité du système (1).

5. Système de multiples sacs (1) selon l'une quelconque des revendications précédentes, dans lequel ledit sac (6, 6', 6") pour l'adhérence dudit produit de composant sanguin à un support biocompatible (100) comprend un support de matériau biocompatible contenu dans celui-ci.

6. Système de multiples sacs (1) selon la revendication précédente, dans lequel ledit matériau biocompatible du support 100 est choisi dans le groupe comprenant :
- un matériau d'origine protéique, qui peut comporter : de la fibroïne, du collagène, de la gélatine, de la rétronectine et d'autres matériaux similaires ;
- des polysaccharides non protéiques, qui peuvent comporter : du chitosane, de l'acide hyaluronique, des alginates, de l'ulvane et d'autres matériaux similaires ;
- des polymères synthétiques non dégradables, qui peuvent comporter : du polyester, des hydrogels et d'autres matériaux similaires ;
- des polymères dégradables, qui peuvent comporter : de l'acide polylactique, de l'acide polyglycolique, de la polycaprolactone et d'autres matériaux similaires ;
- d'autres matériaux qui peuvent comporter : de la céramique (hydroxyapatite).

7. Système de multiples sacs (1) selon l'une quelconque des revendications précédentes 1 à 4, dans lequel ledit support est représenté par la surface interne du sac (6, 6', 6") pour l'adhérence du produit de composant sanguin.

8. Système de multiples sacs (1) selon l'une quelconque des revendications précédentes, dans lequel lesdits sacs (2, 3, 4, 5, 5', 6, 6', 6") sont faits d'un matériau qui ne comprend pas de phtalate de bis(2-éthylhexyle) (DEHP).

9. Système de multiples sacs (1) selon l'une quelconque des revendications précédentes, dans lequel la surface interne desdits sacs (2, 3, 4, 5, 5', 6, 6', 6") est modifiée au moyen de traitements anti-encrassement et/ou anti-adhérence et/ou antithrombotiques.

10. Système de multiples sacs (1) selon revendication 1, dans lequel ladite partie de rupture facilitée (34, 134) comprend une ou plusieurs gravures partielles (35, 36, 35', 36') de l'épaisseur de ladite feuille (31, 131 et/ou 32, 132).

11. Système de multiples sacs (1) selon la revendication précédente, dans lequel lesdites gravures (35, 36, 35', 36') sont mutuellement continues.

12. Système de multiples sacs (1) selon l'une quelconque des revendications précédentes, dans lequel ledit sac (6, 6', 6", 30, 130) pour l'adhérence du produit de composant sanguin comprend en outre un moyen auxiliaire pour l'ouverture du sac.

13. Système de multiples sacs (1) selon la revendication précédente, dans lequel ledit moyen auxiliaire comprend une ou plusieurs parties de préhension (40) pour exercer une force de retenue sur la partie d'ouverture facilitée (34, 134).

14. Système de multiples sacs (1) selon l'une quelconque des revendications précédentes 12 ou 13, dans lequel ledit moyen auxiliaire est couplé aux parties fixes des feuilles supérieure (38, 138) et/ou inférieure (42, 142).

15. Procédé de préparation de composants sanguins, comprenant l'utilisation du système de multiples sacs (1) selon l'une quelconque des revendications précédentes, comprenant les étapes de :
a) soumission d'un échantillon isolé de sang de cordon ombilical compris dans le sac (3) pour la séparation de globules rouges du plasma riche en plaquettes à une centrifugation à faible vitesse réalisée à une vitesse d'environ 220 tr/min pendant une période d'environ 10 minutes, obtenant ainsi un sédiment représenté par des globules rouges et un surnageant représenté par un plasma riche en plaquettes (PRP) ;
b) transfert dudit plasma dans le sac (4) pour la séparation d'un sédiment plaquettaire et d'un surnageant de plasma pauvre en plaquettes ;
c) soumission dudit plasma dans le sac (4) pour la séparation d'un sédiment plaquettaire et d'un surnageant de plasma pauvre en plaquettes à une centrifugation à vitesse élevée réalisée à une vitesse d'environ 2 000 tr/min pendant une période d'environ 15 minutes, obtenant ainsi un plasma pauvre en plaquettes (PPP) et un concentré de plaquettes (PC) ;
d) transfert dudit plasma pauvre en plaquettes (PPP) dans le sac (5) pour la séparation dudit plasma pauvre en plaquettes ;
e) transfert dudit concentré de plaquettes (PC) dans un sac (6) pour l'adhérence d'un produit de composant sanguin à un support biocompatible (100).
